Europäisches Patentamt

European Patent Office

Office européen des brevets

(11) Publication number: **0 141 382 B1**

# EUROPEAN PATENT SPECIFICATION

(45) Date of publication of patent specification: **25.09.91**  (51) Int. Cl.⁵: **C12Q 1/68**, G01T 1/29

(21) Application number: **84112879.6**

(22) Date of filing: **25.10.84**

(54) Method for determining base sequence of DNA or DNA fragment utilizing autoradiography.

(30) Priority: **26.10.83 JP 201231/83**

(43) Date of publication of application:
**15.05.85 Bulletin 85/20**

(45) Publication of the grant of the patent:
**25.09.91 Bulletin 91/39**

(84) Designated Contracting States:
**BE CH DE FR GB LI NL SE**

(56) References cited:
**EP-A- 0 111 154**     **EP-A- 0 115 777**
**EP-A- 0 123 942**     **EP-A- 0 132 621**
**EP-A- 0 138 086**     **WO-A-83/01073**
**GB-A- 2 095 833**     **US-A- 4 236 078**
**US-A- 4 389 670**

(73) Proprietor: **FUJI PHOTO FILM CO., LTD.**
**210 Nakanuma Minamiashigara-shi**
**Kanagawa-ken, 250-01(JP)**

(72) Inventor: **Shiraishi, Hisashi**
**c/o Fuji Photo Film Co., Ltd. No. 210,**
**Nakanuma**
**Minami Ashigara-shi Kanagawa(JP)**
Inventor: **Miyahara, Junji c/o Fuji Photo Film**
**Co., Ltd.**
**No. 798, Miyanodai Kaisei-machi**
**Ashigara-kami-gun Kanagawa(JP)**
Inventor: **Kato, Hisatoyo c/o Fuji Photo Film**
**Co., Ltd.**
**No. 798, Miyanodai Kaisei-machi**
**Ashigara-kami-gun Kanagawa(JP)**

(74) Representative: **Patentanwälte Grünecker,**
**Kinkeldey, Stockmair & Partner**
**Maximilianstrasse 58**
**W-8000 München 22(DE)**

## Description

BACKGROUND OF THE INVENTION

Field of the Invention

The present invention relates to a method for determining the base sequence of DNA or DNA fragment utilizing autoradiography.

Description of the Prior Arts

In molecular biology which has been rapidly developed in recent years, it is essential to obtain genetic information on organisms so as to analyze the functions of organisms or the mechanism of replication. Particularly, it is indispensable to determine the base sequences of nucleic acids such as DNA (or DNA fragment, hereinafter "DNA" may be used to include both DNA and DNA fragment) carrying specific genetic information.

Sanger-Coulson method is known as a representative method for determining the base sequence of DNA. In this method, the base sequence of DNA is determined by ingeniously utilizing synthesis of DNA fragments with DNA synthesis enzyme, gel electrophoresis and autoradiography on the basis of the characteristic structure of DNA that DNA is in the form of a double helix structure consisting of two chain molecules, whose constitutional unit contains any one of four bases: adenine (A), guanine (G), cytosine (C) and thymine (T), the two chain molecules are stabilized through hydrogen bonding between these bases, and that the hydrogen bonding between each two constitutional base units comprises only two combinations, namely, G-C and A-T.

In Sanger-Coulson method, there are some procedures for synthesizing DNA fragments complementary to DNA or DNA fragment to be sequenced (hereinafter referred to as DNA specimen). Basically, single-stranded DNA is used as template, which is incubated with a DNA synthesis enzyme (DNA polymerase) in the presence of mononucleoside triphosphates containing the above four kinds of bases, thus synthesizing (transcribing) DNA fragments of different but discrete lengths and complementary to the DNA specimen. When part of the mononucleoside triphosphates are radioactively labeled, there can be obtained synthesized DNA fragments (in vitro enzymatically synthesized DNA) labeled with a radioactive element.

A mixture of the in vitro enzymatically synthesized DNA fragments is then resolved (developed) on a support medium by electrophoresis, and the support medium is autoradiographed to obtain an autoradiograph of a resolved pattern of the in vitro enzymatically synthesized DNA fragments. The base sequence can be determined in order, starting from the end of the chain molecule according to the visualized autoradiograph. In this way, the sequence for all bases of the DNA specimen can be determined.

Sanger-Coulson method summarized above is described briefly in the following literature: "Reading the genetic information in the original language. A surprising method for sequencing the bases of DNA" written in Japanese by Kin-ichiro Miura, Modern Chemistry, September 1977, pp. 46 - 54 (Tokyo Kagaku Dozin Ltd., Japan).

For obtaining DNA fragments complementary to a DNA specimen in Sanger-Coulson method, a short DNA fragment (referred to as primer) is first bound to the DNA template and DNA is extended from the primer site to synthesize enzymatically in vitro a DNA fragment. Therefore, it is not necessary to determine the base sequence of the primer moiety in analyzing the autoradiograph of the DNA fragments resolved on a support medium. That is, the analysis on relatively short-chain DNA fragments is not required, and resolution may be carried out at such a migration rate that DNA fragments having low molecular weight (i.e., having high migration rate) flow out beyond the lower bottom end. Thus, there is an advantage that a resolution time can be shortened by increasing migration rate in electrophoresis.

Further, a radioactive substance can be incorporated into DNA fragments during the course of synthesizing enzymatically in vitro DNA to obtain DNA fragments with relatively intense radioactivity and hence, the exposure time in autoradiography can be shortened. In addition, there is brought about another advantage that the DNA fragments with relatively intense radioactivity are obtained so that only a small amount of the DNA template is required for this method.

For these reasons, Sanger-Coulson method is a valuable method in the determination of the base sequence of DNA as well as Maxam-Gilbert method utilizing the base-specific cleavage of DNA specimen.

In carrying out the conventional autoradiography for the determination of base sequence of DNA, a radiographic film such as a high-speed X-ray film is placed in layers in combination with a support medium having labeled DNA fragments resolved thereon for a given time so that the film is exposed to the radiation from the support medium. A radiographic intensifying screen is generally employed to enhance the detection sensitivity of autoradiography. Such autoradiography is described, for example, in the following text: Method in Biochemical Experiment, Vol. 6, Method in Tracer Experiment I, pp. 271 - 289, "8. Autoradiography" by Toru Sueyoshi & Akiyo Shigematsu (Tokyo Kagaku Dozin Ltd., 1977).

Therefore, the autoradiography is an important means for obtaining the locational information on DNA fragments resolved on the support medium in the method for determining the base sequence of nucleic acid such as DNA. Nevertheless, such useful autoradiography is not free from several drawbacks in the practical use when applied to the DNA sequence determining method.

As described above, in the conventional autoradiography, a support medium containing radioactively labeled substances is brought into contact in the form of layers with a radiographic film such as a high-speed X-ray film for a given time so that the film is exposed to the radiation and then a visible image indicating the positions of the radioactive substances is obtained.

The primary drawback resides in that the exposure operation requires a long period of time. The exposure operation in the conventional autoradiography for the determination of DNA sequence is usually carried out for several days, and requires at least several tens of hours even when a radiographic intensifying screen is employed. This is because the amount of sample such as DNA fragments fixed to the support medium is small and the radioactively labeled substance (radioactively labeled probe) is generally a nucleic acid partially labeled with e.g. $^{32}P$, so that intense radioactivity is not imparted thereto.

The second drawback resides in that the exposure operation should be carried out usually at a low temperature, for example, a temperature in the range of 0°C to -80°C. This is because a latent image in silver salt of the film formed by exposure to a radiation or light emission, tends to fade at a relatively high temperature such as room temperature, and the so degraded latent image can be no longer so developed as to give a readable image. Further, the silver salt is easily fogged chemically through migration of deleterious ingredients from the support medium carrying the sample to the silver salt layer at such a high temperature. Another reason resides in that the silver salt difficultly forms a latent image at a relatively high temperature such as room temperature even in the case of employing an intensifying screen, because the screen only gives an emission of low intensity.

The third drawback resides in that the exposure operation ought to be done in a dry state to prevent the radiographic film from wetting and being fogged. Generally, the exposure is done after the support medium is dried, or after the support is enclosed with e.g. a synthetic resin wrapping film.

When the image obtained by the autoradiography is fogged as described above, the DNA fragments fractionated on the support medium are hardly located on the obtained image so that the accuracy of base sequence of DNA decreases.

For these reasons, the operations involved in the conventional autoradiography are complicated, whereby the procedure for determining the DNA sequencing is made complicated as a whole.

Other drawbacks of the conventional autoradiography employed in the DNA sequence determining method are given below.

The photosensitive silver salt of the radiographic film is readily influenced by physical irritation and the radiographic film easily produces fogging under application of physical pressure caused by the contact of the film with the hands of operators or the instrument in the exposure operation. Such unfavorable phenomenon also causes lowering in the accuracy of base sequence of DNA. In order to avoid the occurrence of physical fogging on the radiographic film, high skill and caution must be taken in the handling of the film and hence, the determining procedure of DNA sequence is further complicated.

The exposure operation in the conventional autoradiography is conducted over a long period of time as described above so that it is unavoidable that the radiations from natural origin and radioactive impurities incorporated in the support medium in addition to the radioactively labeled substances take part in the exposure of the radiographic film. Thus, the accuracy of the locational information on the labeled substance is lowered. In order to eliminate such interference and to set appropriate exposure conditions, parallel experiments using control samples are generally performed to find out proper exposure time, but such experiments have disadvantages in that the number of experiments involved is increased because such parallel experiments and preliminary experiments for ascertaining appropriate exposure time are required and hence, the operation is made complicated and less economical as a whole.

Further in the determination of DNA sequence utilizing the conventional autoradiography, it is necessary to measure migration distances of resolved DNA fragments on the visualized autoradiograph by eye observation to obtain the locational information for DNA sequence and a long time is taken for such eye measurement.

## SUMMARY OF THE INVENTION

The present inventors have made studies to eliminate the above-mentioned disadvantageous features associated with the method for determining the base sequence of DNA utilizing autoradiography, and found that the above-mentioned disadvantages can be effectively eliminated or reduced by using a stimulable phosphor sheet having a phosphor layer containing a stimulable phosphor as a radiosensitive material in place of the radiographic film.

Accordingly, the present invention provides a method for determining the base sequence of DNA or DNA fragment utilizing autoradiography which comprises steps of:

(1) preparing a mixture of radioactively labeled in vitro enzymatically synthesized DNAs which are complementary to the DNA or DNA fragment to be sequenced;

(2) resolving said mixture of in vitro enzymatically synthesized DNAs on a support medium;

(3) placing said support medium and a stimulable phosphor sheet in layers for a given period of time to cause said phosphor sheet to absorb at least a portion of radiation energy emitted by the radioactively labeled substances on the support medium, exciting said phosphor sheet with an electromagnetic wave to release the radiation energy stored in the phosphor sheet as stimulated emission, and detecting the stimulated emission, to obtain locational information on said radioactively labeled in vitro enzymatically synthesized DNAs; and

(4) determining the base sequence of said DNA or DNA fragment according to the obtained locational information.

The term "locational information" of the radioactively labeled substances on the support medium in the present invention include various information such as the location of the radioactively labeled substances or their aggregate on the support medium, for example, information on the location and shape of the aggregate of the radioactively labeled substances, and on the concentration and the distribution of said radioactively labeled substances or their aggregate. Such information can be obtained singly or in combination.

BRIEF DESCRIPTION OF DRAWINGS

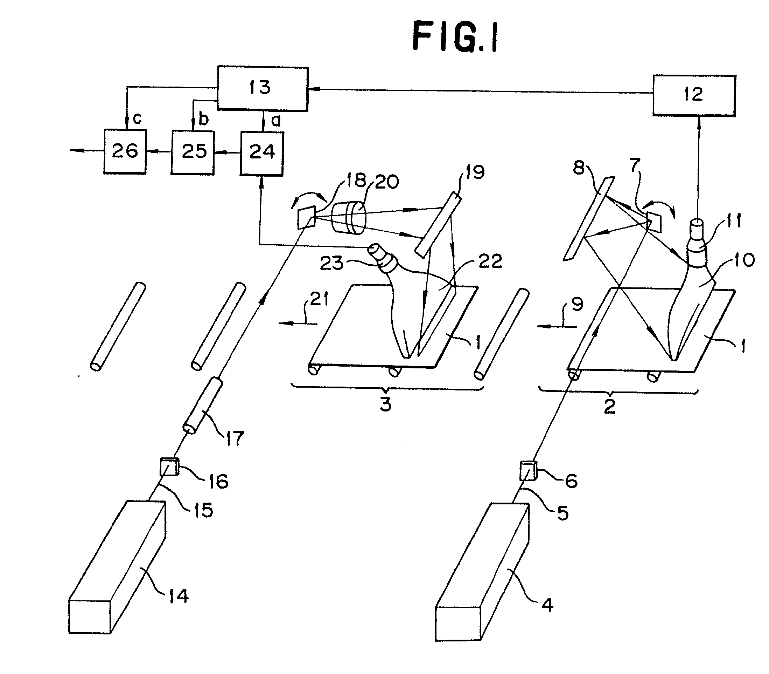

Figure 1 schematically illustrates an embodiment of the read-out system for reading out the locational information on the radioactively labeled substances on a support medium, which is recorded and stored in the stimulable phosphor sheet according to the present invention.

DETAILED DESCRIPTION OF THE INVENTION

The stimulable phosphor sheet used in the present invention is also called a radiation image storage panel and disclosed in, for example, U.S. Patent No. 4,239,968 and thus its general constitution is already known.

The stimulable phosphor sheet comprises a stimulable phosphor, in which said phosphor is capable of absorbing radiation energy having passed through an object or radiated from an object; and releasing the radiation energy stored therein as stimulated emission when said sheet is excited with an electromagnetic wave (stimulating rays) such as visible or infrared rays. The stimulated emission is photoelectrically detected and converted into electric signals which is then reproduced as a visible image on a display device such as CRT or on a recording medium such as a photographic film, or represented as locational information in the form of symbols and/or numerals. The stimulable phosphor sheet can be used repeatedly after erasing the radiation energy remaining therein which is done after being subjected to the read-out operation. Therefore, the use of the stimulable phosphor sheet in the autoradiographic process according to the present invention is very advantageous.

According to the present invention, the stimulable phosphor sheet containing a stimulable phosphor is used in the autoradiography for determination of DNA sequence, in place of the radiographic film or a combination of the film with a radiographic intensifying screen used in the conventional autoradiography. By the use of the stimulable phosphor sheet, not only the exposure time is greatly shortened, but also the accuracy of the detection of the radioactively labeled substances is not lowered even when the exposure is carried out at an ambient temperature or at a temperature thereafound. Therefore, the exposure operation previously taking many hours under chilling, is made simple and hence, the autoradiographic procedure can be greatly simplified.

Moreover, in the case of using the stimulable phosphor sheet as a radiosensitive material, the visualization is not always required to obtain the locational information on the radioactively labeled substances which are stored and recorded on the phosphor sheet, that is, the locational information can be obtained in the desired forms such as a visible image, symbols and/or numerical values and combinations thereof by scanning the phosphor sheet with an electromagnetic wave such as a laser and reading out the

4

locational information on the labeled substances. It is also possible to obtain the required information in the desired various forms by further processing the above-mentioned locational information using an appropriate electric means. Namely, the required information can be obtained not in the form of an image, but in other informational form obtained by further subjecting the above information in the form of an image to certain data processing. In the present invention, it is possible not only that electric signals or digital signals having the locational information on the labeled substances obtained by reading out the stimulable phosphor sheet is reproduced in the form of an image as a pattern of resolved bands, but also that the analysis of said signals is so made as to obtain an information on the base sequence of DNA in question.

Further, by the employment of the stimulable phosphor sheet in the autoradiography as a radiosensitive material, either the chemical fog or the physical fog, both of which are the unavoidable problems in the use of conventional radiographic films, is not produced on the obtained image. This also provides an advantageous feature in the improvement of the accuracy on the determination of the base sequence of DNA and workability of the autoradiography. It is furthermore possible to easily reduce or eliminate the disadvantageous effect which decreases the accuracy such as that caused by the presence of the natural radioactive substances or the radioactivity of impurities in the support medium, by applying a certain electric processing to the locational information stored in the stimulable phosphor sheet.

Accordingly, it becomes possible that analysis for determining the base sequence of DNA according to the locational information on the radioactively labeled substances resolved on the support medium becomes easy and at the same time that the accuracy of the determination of DNA sequence can be improved.

The following illustrates the method for determining the base sequence of DNA or DNA fragment utilizing autoradiography in the present invention by taking a dideoxy terminating method (sometimes called chain termination sequencing method or Sanger-Coulson method) as an example. This sequencing method is one which is extensively used as a simple and highly accurate method.

A single-stranded DNA into which DNA specimen is inserted is first prepared. This is called a DNA template. A short-chain DNA fragment (called primer) complementary to part of the DNA template is prepared and hybridized with the DNA template. Thereafter, the in vitro enzymatically synthesis of DNA complementary to the DNA template is carried out.

The synthesis of DNA is carried out in the following manner.

Four kinds of deoxynucleoside triphosphates (dNTP) containing each of the four bases and a kind of dideoxynucleoside triphosphate (ddNTP) containing a specific base are added to the DNA template having the primer. The primer and template are both incubated together with a DNA polymerase in the presence of the mixture of dNTP and ddNTP to prolong the DNA chain from the primer site of the DNA template. When the dideoxynucleoside triphosphate is incorporated into the DNA template in place of the deoxynucleoside triphosphate in the course of the synthesis, the synthesis reaction of DNA is terminated so that there can be obtained a DNA fragment whose chain is prolonged from the primer site to a specific base site and which is complementary to the DNA specimen. For instance, when dideoxyadenosine triphosphate (ddATP) is used, there is obtained a mixture of DNA fragments of different lengths, each being terminated with adenine (A).

When at least one nucleotide of the above four deoxynucleoside triphosphates is labeled with a radioactive element such as $^{32}$P, there is obtained a radioactively labeled in vitro enzymatically synthesized DNA fragment.

The above synthesis reaction is carried out four times by using each of four kinds of dideoxynucleoside triphosphates to obtain four kinds of in vitro enzymatically synthesized DNAs, each being terminated with a specific base.

Method for in vitro enzymatically synthesizing DNA fragments using dideoxy-nucleoside triphosphates and method for determining the base sequence of DNA according to the obtained in vitro enzymatically synthesized DNA are described in detail in the following literature:

Sanger, F., Nicklen, S., & Coulson, A.R., Proc. Natl. Acad. Sci. USA, 74, pp. 5463 - 5467 (1977).

Various methods for preparing DNA template have been proposed, but a cloning method using bacteriophage M 13 is the simplest one. This method is described, for example, in the following literature:

Schreier, P.H., & Cortese, R., J. Mol. Biol., 129, pp. 169 - 172 (1979).

The resulting four kinds of mixture of in vitro enzymatically synthesized DNA fragments are subjected to gel electrophoresis to obtain a support medium on which each mixture is resolved.

The support medium carrying the labeled in vitro enzymatically synthesized DNA fragments is autoradiographed to determine the base sequence of the DNA specimen.

The characteristic feature of the present invention resides in the autoradiographic process for obtaining the locational information on the radioactively labeled substances resolved on the support medium. In the autoradiographic process for obtaining such locational information, the exposure operation is conducted by placing the support medium and the stimulable phosphor sheet together in layers for a given period of time to cause said phosphor sheet to absorb at least a portion of a radiation radiating from the labeled substances on the support medium.

Generally, the support medium is placed in close contact with the stimulable phosphor sheet during the exposure operation, but it is not always required to place the support medium in close contact with the phosphor sheet and they may be placed adjacent to each other. The support medium is not always required in a dry state, may be in a wet state and may be wrapped e.g. in a polyethylene sheet having such a thickness that does not interfere with transmittance of radiation from the probe.

The exposure time varies depending on the radiation intensity of the radioactively labeled substance contained in the support medium, the amount of said substances, the sensitivity of the stimulable phosphor sheet and the distance between the support medium and the phosphor sheet. The exposure operation must be carried out for a certain period of time, for example, for at least several seconds. In the present invention using the stimulable phosphor sheet as a radiosensitive material, however, the exposure time can be greatly shortened as compared with that required in the case where the conventional radiographic film is used. Further, the precise control of the exposure time is not particularly required, since the locational information on the radioactively labeled substances can be suitably processed in the subsequent read-out operation through applying various electrical processing thereto according to the intensity and distribution of energy stored in the phosphor sheet and the desired information form, for example, by setting the amplification of electric signals to a given value.

There is no specific limitation on the temperature employed for the exposure operation, and it is possible to perform the exposure at an ambient temperature within the range of 10 to 35°C in the autoradiography according to the present invention. If desired, the exposure operation may be, of course, performed at a low temperature of approximately 5°C or lower as in the conventional autoradiography.

The stimulable phosphor sheet suitably employable in the aforementioned autoradiography is composed basically of a support and a phosphor layer comprising a binder and a stimulable phosphor dispersed therein, the phosphor layer being provided on said support. However, in the case that the phosphor layer is of a self-supporting type, the support is not always required.

The stimulable phosphor sheet of the above-described constitution can be prepared, for instance, by the following procedure.

In the first place, phosphor particles and a binder are added to an appropriate solvent (e.g., a lower alcohol, chlorine atom-containing hydrocarbon, ketone, ester, ether), and then they are well mixed to prepare a coating dispersion of the phosphor particles in the binder solution.

Examples of the binder include proteins such as gelatin and synthetic polymers such as polyvinyl acetate, nitrocellulose, polyurethane, polyvinyl alcohol, linear polyester and polyalkyl (meth)acrylate.

The ratio between the binder and the phosphor in the coating dispersion generally is within the range of from 1 : 8 to 1 : 40 (binder : phosphor, by weight).

The coating dispersion is then coated evenly on a support to form a coating layer, and the coating layer is gradually heated to dryness to prepare the phosphor layer on the support. The thickness of the phosphor layer generally ranges from 50 to 500 $\mu$m.

The support may be any one of supports made of various materials which have been known as supports of intensifying papers (i.e., intensifying screens) in the conventional radiography. Examples of the employable supports include films of plastic materials such as cellulose acetate and polyethylene terephthalate, metallic sheets such as aluminum foil, ordinary papers, baryta papers, and resin-coated papers.

On the surface of the support to receive the phosphor layer may be provided one or more of e.g. an adhesive layer, a light-reflecting layer and a light-absorbing layer.

On the surface of the phosphor layer opposite to the surface to face the support, a transparent protective film may be provided to protect the phosphor layer from physical and chemical deterioration. Examples of the material of the protective film include cellulose acetate, polymethyl methacrylate, polyethylene terephthalate and polyethylene. The thickness of the transparent protective film generally ranges from 0.1 to 20 $\mu$m.

Moreover, the surface of the stimulable phosphor sheet may be treated, for instance, hydrophilically, if desired.

The stimulable phosphor contained in the stimulable phosphor sheet utilized in the present invention gives stimulated emission when excited with stimulating rays after exposure to a radiation. From the viewpoint of practical use, the stimulable phosphor is desired to give stimulated emission in the wavelength region of 300 - 500 nm when excited with stimulating rays in the wavelength region of 400 - 850 nm.

Examples of the stimulable phosphor employable in the radiation image storage panel of the present invention include:

SrS:Ce,Sm, SrS:Eu,Sm, $ThO_2$:Er, and $La_2O_2S$:Eu,Sm, as described in U.S. Patent No. 3,859,527;

6

ZnS:Cu,Pb, $BaO \cdot xAl_2O_3:Eu$, in which x is a number satisfying the condition of $0.8 \leq x \leq 10$, and $M^{2+}O \cdot xSiO_2:A$, in which $M^{2+}$ is at least one divalent metal selected from the group consisting of Mg, Ca, Sr, Zn, Cd and Ba, A is at least one element selected from the group consisting of Ce, Tb, Eu, Tm, Pb, Tl, Bi and Mn, and x is a number satisfying the condition of $0.5 \leq x \leq 2.5$, as described in U.S. Patent No. 4,326,078;

$(Ba_{1-x-y},Mg_x,Ca_y)FX:aEu^{2+}$, in which X is at least one element selected from the group consisting of Cl and Br, x and y are numbers satisfying the conditions of $0 < x+y \leq 0.6$, and $xy \neq 0$, and a is a number satisfying the condition of $10^{-6} \leq a \leq 5 \times 10^{-2}$, as described in Japanese Patent Provisional Publication No. 55(1980)-12143;

LnOX:xA, in which Ln is at least one element selected from the group consisting of La, Y, Gd and Lu, X is at least one element selected from the group consisting of Cl and Br, A is at least one element selected from the group consisting of Ce and Tb, and x is a number satisfying the condition of $0 < x < 0.1$, as described in U.S. Patent No. 4,236,078;

$(Ba_{1-x},M^{II}_x)FX:yA$, in which $M^{II}$ is at least one divalent metal selected from the group consisting of Mg, Ca, Sr, Zn and Cd, X is at least one element selected from the group consisting of Cl, Br and I, A is at least one element selected from the group consisting of Eu, Tb, Ce, Tm, Dy, Pr, Ho, Nd, Yb and Er, and x and y are numbers satisfying the conditions of $0 \leq x \leq 0.6$ and $0 \leq y \leq 0.2$, respectively, as described in U.S. Patent No. 4,239,968;

$M^{II}FX \cdot xA:yLn$, in which $M^{II}$ is at least one element selected from the group consisting of Ba, Ca, Sr, Mg, Zn and Cd; A is at least one compound selected from the group consisting of BeO, MgO, CaO, SrO, BaO, ZnO, $Al_2O_3$, $Y_2O_3$, $La_2O_3$, $In_2O_3$, $SiO_2$, $TiO_2$, $ZrO_2$, $GeO_2$, $SnO_2$, $Nb_2O_5$,$Ta_2O_5$ and $ThO_2$; Ln is at least one element selected from the group consisting of Eu, Tb, Ce, Tm, Dy, Pr, Ho, Nd, Yb, Er, Sm and Gd; X is at least one element selected from the group consisting of Cl, Br and I; and x and y are numbers satisfying the conditions of $5 \times 10^{-5} \leq x \leq 0.5$ and $0 < y \leq 0.2$, respectively, as described in Japanese Patent Provisional Publication No. 55(1980)-160078;

$(Ba_{1-x},M^{II}_x)F_2 \cdot aBaX_2:yEu,zA$, in which $M^{II}$ is at least one element selected from the group consisting of Be, Mg, Ca, Sr, Zn and Cd; X is at least one element selected from the group consisting of Cl, Br and I; A is at least one element selected from the group consisting of Zr and Sc; and a, x, y and z are numbers satisfying the conditions of $0.5 \leq a \leq 1.25$, $0 \leq x \leq 1$, $10^{-6} \leq y \leq 2 \times 10^{-1}$, and $0 < z \leq 10^{-2}$, respectively, as described in Japanese Patent Provisional Publication No. 56(1981)-116777;

$(Ba_{1-x},M^{II}_x)F_2 \cdot aBaX_2:yEu,zB$, in which $M^{II}$ is at least one element selected from the group consisting of Be, Mg, Ca, Sr, Zn and Cd; X is at least one element selected from the group consisting of Cl, Br and I; and a, x, y and z are numbers satisfying the conditions of $0.5 \leq a \leq 1.25$, $0 \leq x \leq 1$, $10^{-6} \leq y \leq 2 \times 10^{-1}$, and $0 < z \leq 2 \times 10^{-1}$, respectively, as described in Japanese Patent Provisional Publication No. 57(1982)-23673;

$(Ba_{1-x},M^{II}_x)F_2 \cdot aBaX_2:yEu,zA$, in which $M^{II}$ is at least one element selected from the group consisting of Be, Mg, Ca, Sr, Zn and Cd; X is at least one element selected from the group consisting of Cl, Br and I; A is at least one element selected from the group consisting of As and Si; and a, x, y and z are numbers satisfying the conditions of $0.5 \leq a \leq 1.25$, $0 \leq x \leq 1$, $10^{-6} \leq y \leq 2 \times 10^{-1}$, and $0 < z \leq 5 \times 10^{-1}$, respectively, as described in Japanese Patent Provisional Publication No. 57(1982)-23675;

$M^{III}OX:xCe$, in which $M^{III}$ is at least one trivalent metal selected from the group consisting of Pr, Nd, Pm, Sm, Eu, Tb, Dy, Ho, Er, Tm, Yb, and Bi; X is at least one element selected from the group consisting of Cl and Br; and x is a number satisfying the condition of $0 < x < 0.1$, as described in Japanese Patent Provisional Publication No. 58(1983)-69281;

$Ba_{1-x}M_{x/2}L_{x/2}FX:yEu^{2+}$, in which M is at least one alkali metal selected from the group consisting of Li, Na, K, Rb and Cs; L is at least one trivalent metal selected from the group consisting of Sc, Y, La, Ce, Pr, Nd, Pm, Sm, Gd, Tb, Dy, Ho, Er, Tm, Yb, Lu, Al, Ga, In and Tl; X is at least one halogen selected from the group consisting of Cl, Br and I; and x and y are numbers satisfying the conditions of $10^{-2} \leq x \leq 0.5$ and $0 < y \leq 0.1$, respectively, as described in U.S. Patent Application No. 497,805;

$BaFX \cdot xA:yEu^{2+}$, in which X is at least one halogen selected from the group consisting of Cl, Br and I; A is at least one fired product of a tetrafluoroboric acid compound; and x and y are numbers satisfying the conditions of $10^{-6} \leq x \leq 0.1$ and $0 < y \leq 0.1$, respectively, as described in U.S. Patent Application No. 520,215;

$BaFX \cdot xA:yEu^{2+}$, in which X is at least one halogen selected from the group consisting of Cl, Br and I; A is at least one fired product of a hexafluoro compound selected from the group consisting of monovalent and divalent metal salts of hexafluoro silicic acid, hexafluoro titanic acid and hexafluoro zirconic acid; and x and y are numbers satisfying the conditions of $10^{-6} \leq x \leq 0.1$ and $0 < y \leq 0.1$, respectively, as described in U.S Patent Application No. 502,648;

$BaFX \cdot xNaX':aEu^{2+}$, in which each of X and X' is at least one halogen selected from the group

consisting of $Cl$, Br and I; and x and a are numbers satisfying the conditions of $0 < x \leq 2$ and $0 < a \leq 0.2$, respectively, as described in Japanese Patent Provisional Publication No. 59(1984)-56479;

$M^{II}FX \cdot xNaX':yEu^{2+}:zA$, in which $M^{II}$ is at least one alkaline earth metal selected from the group consisting of Ba, Sr and Ca; each of X and X' is at least one halogen selected from the group consisting of $Cl$, Br and I; A is at least one transition metal selected from the group consisting of V, Cr, Mn, Fe, Co and Ni; and x, y and z are numbers satisfying the conditions of $0 < x \leq 2$, $0 < y \leq 0.2$ and $0 < z \leq 10^{-2}$, respectively, as described in U.S. Patent Application No. 535,928; and

$M^{II}FX \cdot aM^{I}X' \cdot bM^{III}X''_2 \cdot cM^{III}X'''_3 \cdot xA:yEu^{2+}$, in which $M^{II}$ is at least one alkaline earth metal selected from the group consisting of Ba, Sr and Ca; $M^{I}$ is at least one alkali metal selected from the group consisting of Li, Na, K, Rb and Cs; $M^{III}$ is at least one divalent metal selected from the group consisting of Be and Mg; $M^{III}$ is at least one trivalent metal selected from the group consisting of $Al$, Ga, In and $Tl$; A is at least one metal oxide; X is at least one halogen selected from the group consisting of $Cl$, Br and I; each of X', X'' and X''' is at least one halogen selected from the group consisting of F, $Cl$, Br and I; a, b and c are numbers satisfying the conditions of $0 \leq a \leq 2$, $0 \leq b \leq 10^{-2}$, $0 \leq c \leq 10^{-2}$ and $a+b+c \geq 10^{-6}$; and x and y are numbers satisfying the conditions of $0 < x \leq 0.5$ and $0 < y \leq 0.2$, respectively, as described in U.S. Patent Application No. 543,326.

The above-described stimulable phosphors are given by no means to restrict the stimulable phosphor employable in the present invention. Any other phosphor can be also employed, provided that the phosphor gives stimulated emission when excited with stimulating rays after exposure to a radiation.

Detailed description on the stimulable phosphor sheet and the exposing procedure employable in the present invention is given in Japanese Patent Application No. 57(1982)-193418 (U.S. Patent Application No. 549,417 and European Patent Application 83 110 984.8).

A method for reading out or detecting the locational information on the radioactively labeled substances which is stored in the stimulable phosphor sheet is described below briefly, referring to an embodiment of a read-out system shown in Figure 1 of the accompanying drawings.

Figure 1 schematically illustrates an embodiment of the read-out system comprising a preliminary read-out section 2 for preliminarily reading out the two-dimensional information on the location of the radioactively labeled substances stored (or recorded) in the stimulable phosphor sheet 1 (stimulable phosphor sheet may be hereinafter referred to as "phosphor sheet"), and a final read-out section 3 for finally reading out the desired locational information on the radioactively labeled substance stored in the phosphor sheet 1.

In the preliminary read-out section 2, the preliminary read-out operation is carried out in the following manner.

Laser beam 5 generated by a laser source 4 first passes through a filter 6 to cut off a light beam in the wavelength region corresponding to the wavelength region of stimulated emission to be emitted from the phosphor sheet 1 in response to stimulation with the laser beam 5. The laser beam 5 is subsequently deflected by a beam deflecter 7 such as a galvanometer mirror, and reflected by a plane reflecting mirror 8. The deflected beam then impinges upon the phosphor sheet 1. The laser source 4 used herein is so selected as to avoid overlapping of the wavelength region of the laser beam 5 with the main wavelength region of the stimulated emission to be emitted from the phosphor sheet 1.

The phosphor sheet 1 is transferred in the direction along the arrow 9 under the irradiation of the above-mentioned deflected laser beam. Therefore, the whole surface of the phosphor sheet 1 is subjected to the irradiation of the deflected laser beam. The power of the laser beam 5 employed in the preliminary read-out section is adjusted to be lower than the power of the laser beam to be employed in the final read-out section by controlling the output of the laser source 4, the beam diameter of the laser beam 5, the scanning speed of the laser beam 5, and the transferring speed of the phosphor sheet 1.

When irradiated with the above-mentioned laser beam, the phosphor sheet 1 gives stimulated emission having the emission intensity proportional to the radiation energy stored (or recorded) therein. The emission then enters into a light guiding sheet 10 for the preliminary read-out. The light guiding sheet 10 has a linear edge face for receiving the emission, and the edge face is so positioned in the vicinity of the phosphor sheet as to correspond to the scanning line on the phosphor sheet 1. The exit of the light guiding sheet 10 is in the form of a ring and is connected to an light-receiving face of a light detector 11 such as a photomultiplier. The light guiding sheet 10 is made, for instance, by processing a sheet of a transparent thermoplastic resin such as a polyacrylic synthetic resin, and so constituted that the emission introduced from the linear edge face is transmitted to the exit under repeated total reflection within the sheet 10. The stimulated emission from the phosphor sheet 1 is guided in the interior of the light guiding sheet 10 to the exit, and received by the light detector 11.

On the light-receiving face of the light detector 11 is provided a filter which allows only the light of wavelength region of the stimulated emission to pass through and cuts off the light of the wavelength region of the stimulating rays (laser beam) so as to detect only the stimulated emission. The stimulated emission detected by the light detector 11 is converted to an electric signal, amplified in an amplifier 12 and transmitted to the output. The stored information output from the amplifier 12 is supplied to a control circuit 13 of the final read-out section 3. The control circuit 13 provides an amplification degree setting value a, a scale factor setting value b, and an image processing condition setting value c, for obtaining a well readable image having uniform concentration and contrast regardless of variation of the detected information.

The phosphor sheet 1 having been subjected to the preliminary read-out in the above-described manner is then transferred to the final read-out section 3.

In the final read-out section 3, the following read-out operation is performed.

The laser beam 15 generated by a laser source 14 for the final read-out passes through a filter 16 having the same function as that of the above-mentioned filter 6, and then the beam diameter is precisely adjusted in a beam expander 17. Subsequently, the laser beam is deflected by a beam deflector 18 such as a galvanometer mirror, and reflected by a plane reflection mirror 19. The deflected beam then impinges one-dimensionally upon the phosphor sheet 1. Between the beam deflector 18 and the plane reflection mirror 19 a f$\theta$ lens 20 is provided so that the beam speed is continuously kept constant while the deflected laser beam scans the phosphor sheet 1.

The phosphor sheet 1 is transferred in the direction along the arrow 21 under the irradiation with the above-mentioned deflected laser beam. Accordingly, the whole surface of the phosphor sheet is subjected to the irradiation in the same manner as in the preliminary read-out operation.

When irradiated with the above-mentioned laser beam, the phosphor sheet 1 gives the stimulated emission in proportion to the radiation energy stored therein in the same manner as in the preliminary read-out operation. The emission then enters into a light guiding sheet 22 for the final read-out. The light guiding sheet 22 for the final read-out is made of the same material and has the same constitution as the light guiding sheet 10 employed for the preliminary read-out. The stimulated emission received is guided in the interior of the light guiding sheet 22 up to the exit under repeated total reflection, and then received by a light detector 23. On the light-receiving face of the light detector 23 is provided a filter which allows only the light of wavelength region of the stimulated emission to pass through and cuts off the light of the wavelength region of the stimulating rays (laser beam) so as to detect only the stimulated emission.

The stimulated emission detected by the light detector 23 is converted to an electric signal, amplified to an electric signal adjusted to an appropriate level in an amplifier 24 according to the aforementioned amplification degree setting value a and transmitted to an A/D converter 25. The adjusted electric signal is then converted to a digital signal in the A/D converter 25 according to an appropriate scale factor defined by the scale factor setting value b, and then the digital signal is input into a signal processing circuit 26. In the signal processing circuit 26, the digital signal is processed according to the image processing condition setting value c for obtaining a well readable visible image having appropriate density and contrast regardless of variation of the detected information. If desired, the signal thus processed is then transmitted to a recording device (not shown) via a data preserving means such as a magnetic tape.

Various recording devices based on various systems can be employed for the above described purpose, for instance, a device for visualizing optically by scanning a photosensitive material with laser beam, etc., a display means for visualizing electrically on CRT, etc., a means for printing a radiation image displayed on CRT by means of video printer, and a means for visualizing on heat-sensitive recording material using thermic rays.

The recording device used in the present invention is not restricted to the visualizing devices such as mentioned above, and the two-dimensional information on the location of the radioactively labeled substance can be recorded, for example, in the form of numerals and/or symbols.

In the above description on the method for reading out the locational information on the radioactively labeled substances stored in the stimulable phosphor sheet, a read-out operation involving both the preliminary read-out operation and the final read-out operation has been given. However, the read-out operation employable in the present invention is not limited to the above-described embodiment. For instance, the preliminary read-out operation may be omitted if the content of the radioactive substances on the support medium and an adequate exposure time for the support medium is previously known.

Further, other suitable methods than the above-mentioned embodiments may be used for reading out the locational information of the radioactively labeled substances stored in the stimulable phosphor sheet.

The base sequence of DNA can be determined according to the thus obtained information on the location of the radioactively labeled substances on the support medium. Namely, the positions (migration distances) of the radioactively labeled substances are compared with one another among resolution tracks of the in vitro enzymatically synthesized DNA fragment mixtures (base-specifically synthesized DNA), each mixture being terminated with a specific base selected from A, G, T and C. The positions are arranged in order of longer migration distance and each terminal base of the synthesized DNA fragments is assigned, thus determining the base sequence of DNA.

Since the single-stranded in vitro enzymatically synthesized DNA is complementary to one strand of the double-stranded DNA specimen, the above determination of the base sequence of the in vitro enzymatically synthesized DNA means determination of base sequence for one strand of the double-stranded DNA specimen. The base sequence for all the double-stranded DNA specimen can be determined on the basis of the one strand whose base sequence is determined. Alternatively, when the DNA specimen is single-stranded DNA, the determination of the base sequence of the in vitro enzymatically synthesized DNA means determination of base sequence for one complementary to the DNA specimen, so that the single-stranded DNA specimen can be determined based on the resulting base sequence.

While there has been described the method for determining the base sequence of DNA by obtaining the two-dimensional information on the location of the radioactively labeled substances on the support medium utilizing autoradiography, it will be understood that the present invention is not to be limited to the method wherein the autoradiograph is visualized on the radiographic film. For example, it is possible that the locational information is represented in the form of symbols or numerals and the base sequence of DNA is determined according to the resulting digital values such as numerals or symbols.

Further, it is also possible to directly obtain the base sequence of the DNA specimen by applying a suitable signal processing for determining the base sequence of DNA to the resulting digital signals. Such signal processing methods are described in, for example, Japanese Patent Application No. 58(1983)-1326, No. 58(1983)-1327, No. 58(1983)-1328 and No. 58(1983)-1329 (corresponding to U.S. Patent Application No. 568,877 and No. 568,906).

The above descriptions have been directed to the method for determining the base sequence of DNA utilizing the dideoxy terminating method, developed by Sanger and Coulson, to illustrate the present invention, but are not to be construed as limiting the present invention in any way. For example, the present invention is applicable to other methods such as the "plus and minus" method, also developed by Sanger and Coulson. Further, the present invention is not to be limited to these Sanger-Coulson methods. The present invention can be applied to any methods capable of using in vitro enzymatically synthesized DNA and determining the base sequence of DNA by autoradiographic technique.

Examples of the support medium on which the in vitro enzymatically synthesized DNA is resolved include support mediums for electrophoresis such as e.g. agarose gel and polyacrylamide gel, support mediums for thin layer chromatography such as e.g. silica gel; and support mediums for paper chromatography such as e.g. filter paper.

Examples of the radioisotope for the nucleotide to radioactively label the in vitro enzymatically synthesized DNA include $^{32}P$, $^{3}H$, $^{14}C$ and $^{35}S$.

The present invention will be further illustrated by the following examples in which the invention is performed according to the afore-described dideoxy terminating method of Sanger and Coulson. The base sequence of DNA specimen (plasmid pBR 322) used in the examples was already known, and the results on the base sequence in the examples were evaluated in comparison with the known data.

The stimulable phosphor sheet used in the example was prepared by the following method.

To a mixture of a powdery divalent europium activated barium fluorobromide stimulable phosphor (BaFBr:Eu$^{2+}$) and a linear polyester resin were added successively methyl ethyl ketone and nitrocellulose (nitrification degree: 11.5 %) to prepare a dispersion containing the phosphor particles. Subsequently, tricresyl phosphate, n-butanol and methyl ethyl ketone were added to the resulting dispersion. The mixture was thoroughly stirred by means of a propeller agitator to obtain a homogeneous coating dispersion having a viscosity of 25 - 35 PS (at 25°C) and containing the phosphor particles uniformly dispersed therein and the binder and the phosphor in a mixing ratio of 1 : 25 by weight.

The coating dispersion was applied to a polyethylene terephthalate sheet containing carbon black (250 $\mu$m in thickness, support) placed horizontally on a glass plate. The application of the coating dispersion was carried out by using a doctor blade. After the application, the support having a layer of the coating dispersion was placed in a dryer and the temperature within the dryer was gradually elevated from 25 to 100°C to dry the layer. Thus, a phosphor layer of 300 $\mu$m in thickness was formed on the support.

On the phosphor layer was placed a transparent polyethylene terephthalate film (12 $\mu$m in thickness, provided with a polyester adhesive layer on one side) to combine the film and the phosphor layer with the adhesive layer. Thus, a stimulable phosphor sheet comprising a support, a phosphor layer and a protective film was prepared.

EXAMPLE 1

(1) Preparation of DNA template containing DNA specimen

Plasmid DNA of E. coli (pBR 322) was cleaved by the use of restriction enzyme Hind-III by the known method to obtain 100 ng. of DNA fragment. To the DNA fragment was added 20 ng. of bacteriophage M13 mp8 (N.4501, available from Amersham) which was previously treated with the same restriction enzyme Hind-III as DNA vector. The insertion was effected by using T4 DNA ligase by the known method. E. coli K12 strain which was in logarithmic phase was then infected with the resulting recombinant DNA and incubated overnight.

100 ml of 2xTY medium (containing 16 g. of bactotryptone, 10 g. of yeast extract and 5 g. of sodium chloride per litre) was inoculated with 1 ml of the resulting E. coli culture (colorless plaque) containing the recombinant DNA (M13) by using a cocktail stick made of wood. The inoculated medium was transferred to a culture tube, shaken at 37°C for 5 hours and centrifuged. The supernatant was recovered carefully to remove the E. coli.

This supernatant was added to a solution containing 20 % PEG (polyethylene glycol 6000) and 2.5 M sodium chloride, followed by well stirring. The PEG layer was then removed by centrifugation. To a pellet-form solid which remained at bottom were added 100 μl of TE buffer solution (containing 10 mM tris-HCl solution and 1 mM of EDTA, pH 8.0) and subsequently 50 μl of phenol saturated with TE buffer solution, followed by stirring thoroughly and leaving to stand at room temperature for 15 minutes. The resulting suspension was centrifuged and the supernatant was transferred to 10 μl of 3 M sodium acetate solution, and 250 μl of ethanol was added thereto. The mixture was left to stand at -20°C overnight and then centrifuged. The resulting precipitate was washed with cold ethanol and dried. The dried product was dissolved in 50 μl of TM buffer solution to obtain a DNA template solution containing DNA specimen.

(2) Hybridization of DNA template with primer

5 μl of the above DNA template, 1 μl of M13 primer (N.4502, available from Amersham) and 1.5 μl of Klenow reaction buffer solution (containing 10 mM magnesium chloride and 10 mM tris, pH 8.5) were added to 2.5 μl of distilled water. The mixture was cultured at a temperature of 55 - 60°C for 2 hours to obtain primer-hybridized DNA template.

(3) Synthesis reaction

2 μl of [α-$^{32}$P] dATP (specific activity: 800 Ci/m mole, PB 10384, available from Amersham) and 1 μl - (1 unit) of Klenow fragment (DNA polymerase I) were added to the above primer-hybridized DNA template. 2.5 μl of the resulting mixture was charged in each of four test tubes. 2 μl of a reactive mononucleotide mixture solution (A#, C#, G#, or T#) composed of ingredient or ingredients given in the following Table 1, was added to each test tube, and mixing was conducted to obtain four kinds of mixture solutions. Each of these mixture solutions was reacted at room temperature for 15 minutes. 2 μl of chase reaction solution (0.5 mM mixture solution of four kinds of monodeoxynucleoside triphosphates) was added to each of the above mixture solutions, and reaction was done further for 15 minutes. To the reacted solution was added 4 μl of formamide which was previously subjected to an ion exchange treatment, to terminate the reaction.

## Table 1

|  | A# | C# | G# | T# |
|---|---|---|---|---|
| 1 x TE buffer solution | 20 | 20 | 20 | 20 |
| 0.5 mM dCTP | 20 | 1 | 20 | 20 |
| 0.5 mM dGTP | 20 | 20 | 1 | 20 |
| 0.5 mM dTTP | 20 | 20 | 20 | 1 |
| 0.1 mM ddATP | 80 | – | – | – |
| 0.1 mM ddCTP | – | 60 | – | – |
| 0.3 mM ddGTP | – | – | 60 | – |
| 0.5 mM ddTTP | – | – | – | 60 |

Thus, the synthesis was carried out using the four kinds of A# to T# solutions, and there were obtained adenine (A)-specifically synthesized DNA, cytosine (C)-specifically synthesized DNA, guanine (G)-specifically synthesized DNA and thymine (T)-specifically synthesized DNA.

(4) Preparation of gel for electrophoresis

5.7 g. of acrylamide, 0.3 g. of N,N'-methylene bis-acrylamide, 42 g. of urea and 10 mℓ of 1.0 M tris-borate buffer solution (pH 8.3) containing 20 mM EDTA were dissolved in distilled water to prepare 100 mℓ of a gel solution. Nitrogen gas was passed into the gel solution to remove oxygen, and 600 $\mu\ell$ of 10 % aqueous ammonium persulfate solution and 100 $\mu\ell$ of TEMED (tetramethylethylenediamine) catalyst were added to the gel solution. Thus treated gel solution was poured into a mold (0.5 mm x 200 mm x 400 mm) which was constructed with sufficiently cleaned two glass plates of 3 mm in thickness. A slot former for forming four slots (0.5 mm x 15 mm x 20 mm each) was placed at the upper end on the gel solution, and the gel solution was then allowed to stand overnight to complete the gelation. Thus, a gel having four slots for electrophoresis was prepared.

(5) Electrophoresis

The gel prepared as above was placed in an electrophoresis apparatus.

Subsequently, (A)-specifically synthesized DNA, (C)-specifically synthesized DNA, (D)-specifically synthesized DNA, and (T)-specifically synthesized DNA were introduced in the amount of 2 $\mu\ell$ into the first slot, the second slot, the third slot and the fourth slot, respectively, by using a micropipette. The introduction of the synthesized DNAs into the slots was conducted after the slots were washed by blowing an electrode solution to the slots and removing urea eluting from the gel.

The electrophoresis on the samples was then carried out by applying DC voltage at 2000 V/40 cm to the gel. The electrophoresis was continued until the marker dye BPB (Bromophenol Blue) previously added to the DNA sample reached the position which was situated at a height of about 3 cm from the bottom of the gel. The power source was then shut off and the gel was removed from the electrophoresis apparatus.

One glass plate was removed from the mold, and the gel was immersed several times in 10 % aqueous acetic acid to fix DNA, said immersion being conducted each time for 15 minutes with a fresh aqueous acetic acid. The resulting gel was transferred onto a filter paper and heated to dryness on the paper under vacuum.

(6) Autoradiography

The dry gel obtained as above and the stimulable phosphor sheet were placed together in layers in a cassette, and the exposure was performed at room temperature (approx. 20°C) for 10 minutes. The phosphor sheet was then placed in the read-out system of Figure 1 to read out an autoradiograph of the four resolved tracks indicating the location of the radioactively labeled substances on the gel support medium, which was recorded and stored in the phosphor sheet. The read-out locational information was stored in a memory device in the form of digital signals.

Visualization was applied to the obtained digital information to give a clear image. Upon comparison, it was confirmed that the thus-obtained visualized autoradiograph well agreed with the known autoradiograph of the same plasmid DNA of E. Coli (pBR 322).

(7) Determination of base sequence of DNA

The distance from the bottom of the slot to each migration band (corresponding to the radioactively labeled DNA fragment fractionated depending on the molecular weight) was measured on the migration track of each slot of the resulting autoradiograph. The bands were arranged in order of longer distance to give a progression $\{X^y_i\}$, wherein X is a migration distance, $\underline{y}$ is a symbol corresponding to the slot of different base from each other (in this case, the first slot is A, the second slot is C, the third slot is G and the fourth slot is T) and i is the order arranged as above.

The progression $\{X^y_i\}$ was represented with the symbol $\underline{y}$ corresponding to the slot, thus obtaining the base sequence of DNA.

The base sequence obtained as above well agreed with the known base sequence of the same plasmid DNA of Escherichia coli (pBR 322).

It is apparent from the results that the determination of the base sequence of pBR 322 cloned by bacteriophage M13 according to the dideoxy sequencing method can be satisfactorily attained by the autoradiography at room temperature for a short period of time using the stimulable phosphor sheet.

Comparison Example 1

The exposure procedure using the dry gel obtained through the electrophoresis and the stimulable phosphor sheet as described in "(6) Autoradiography" of Example 1 was performed by using X-ray film (RX-type, produced by Fuji Photo Film Co., Ltd., Japan) in place of the stimulable phosphor sheet. The dry gel and the X-ray film were placed together in layers in a conventional X-ray cassette and kept under the same conditions as those of Example 1, namely, at room temperature for 10 min. Then the film was developed, but a well readable autoradiograph was not obtained.

Alternatively, the above-described exposure procedure was performed according to the conventional autoradiography employed in the dideoxy sequencing method, at -80°C for 1,200 min, and the X-ray film was developed. A visualized autoradiograph was obtained as the same clear image as that obtained in Example 1 and agreed therewith.

**Claims**

1.  A method for determining the base sequence of DNA or DNA fragment utilizing autoradiography which comprises the steps of:

    (1) preparing a mixture of radioactively labeled in vitro enzymatically synthesized DNAs which are complementary to the DNA or DNA fragment to be sequenced;

    (2) resolving said mixture of in vitro enzymatically synthesized DNAs on a support medium;

    (3) placing said support medium and a stimulatable phosphor sheet in layers for a given period of time to cause said phosphor sheet to absorb at least a portion of radiation energy emitted by the radioactively labeled substances on the support medium, exciting said phosphor sheet with an electromagnetic wave to release the radiation energy stored in the phosphor sheet as stimulated emission, and detecting the stimulated emission, to obtain locational information on said radioactively labeled in vitro enzymatically synthesized DNAs; and

    (4) determining the base sequence of said DNA or DNA fragment according to the obtained locational information.

2.  The method for determining the base sequence of DNA or DNA fragment utilizing autoradiography as claimed in claim 1, wherein said in vitro enzymatically synthesized DNAs in the step (1) are those prepared by the dideoxy terminating method.

3. The method for determining the base sequence of DNA or DNA fragment utilizing autoradiography as claimed in any one of claims 1 or 2, wherein said mixture of in vitro enzymatically synthesized DNAs in the step (2) is resolved by gel electrophoresis.

4. The method for determining the base sequence of DNA or DNA fragment utilizing autoradiography as claimed in any one of claims 1 or 2, wherein said stimulatable phosphor sheet in the step (3) is excited by sequentially scanning it with an electromagnetic wave.

5. The method for determining the base sequence of DNA or DNA fragment utilizing autoradiography as claimed in any one of claims 1 or 2, wherein said locational information on the radioactively labeled substances in the step (3) is obtained as an image.

6. The method for determining the base sequence of DNA or DNA fragment utilizing autoradiography as claimed in any one of claims 1 or 2, wherein said locational information on the radioactively labeled substances in the step (3) is obtained in the form of symbols and/or numerals.

7. The method for determining the base sequence of DNA or DNA fragment utilizing autoradiography as claimed in any one of claims 1 or 2, wherein said stimulatable phosphor sheet comprises a support, a phosphor layer comprising a stimulable phosphor dispersed in a binder, and a protective film.

**Revendications**

1. Procédé de détermination de la séquence de base d'un ADN ou fragment d'ADN utilisant l'autoradiographie, qui comprend les étapes de:
   (1) préparation d'un mélange d'ADN synthétisés enzymatiquement in vitro et radioactivement marqués, qui sont complémentaires de l'ADN ou fragment d'ADN à séquencer;
   (2) division dudit mélange d'ADN synthétisés enzymatiquement in vitro sur un milieu support;
   (3) disposition dudit milieu support et d'une feuille de matière phosphorescente stimulable en couches pendant une durée donnée pour que ladite feuille de matière phosphorescente absorbe au moins une partie de l'énergie de rayonnement émise par les substances à marquage radioactif sur le milieu support, en excitant ladite feuille de matière phosphorescente avec une onde électromagnétique pour libérer l'énergie de rayonnement emmagasinée dans la feuille de matière phosphorescente sous la forme d'une émission stimulée, et en détectant l'émission stimulée, afin d'obtenir une information en matière de localisation sur lesdits ADN synthétisés enzymatiquement in vitro et radioactivement marqués; et
   (4) détermination de la séquence de base dudit ADN ou fragment d'ADN selon l'information obtenue en matière de localisation.

2. Procédé de détermination de la séquence de base de l'ADN ou fragment d'ADN utilisant l'autoradiographie selon la revendication 1, dans lequel lesdits ADN synthétisés enzymatiquement in vitro dans l'étape (1) sont ceux qui sont préparés par le procédé de la terminaison didésoxy.

3. Procédé de détermination de la séquence de base d'un ADN ou fragment d'ADN utilisant l'autoradiographie selon l'une quelconque des revendications 1 ou 2, dans lequel ledit mélange d'ADN synthétisés enzymatiquement in vitro dans l'étape (2) est divisé par électrophorèse sur gel.

4. Procédé de détermination de la séquence de base d'un ADN ou fragment d'ADN utilisant l'autoradiographie selon l'une quelconque des revendications 1 ou 2, dans lequel ladite feuille de matière phosphorescente stimulable dans l'étape (3) est excitée par balayages successifs avec une onde électromagnétique.

5. Procédé de détermination de la séquence de base d'un ADN ou fragment d'ADN utilisant l'autoradiographie selon l'une quelconque des revendications 1 ou 2, dans lequel ladite information en matière de localisation sur les substances à marquage radioactif dans l'étape (3) est obtenue sous la forme d'une image.

14

6. Procédé de détermination de la séquence de base d'un ADN ou fragment d'ADN utilisant l'autoradiographie selon l'une quelconque des revendications 1 ou 2, dans lequel ladite information de localisation sur les substances radioactivement marquées dans l'étape (3) est obtenue sous forme de symboles et/ou de chiffres.

7. Procédé de détermination de la séquence de base d'un ADN ou fragment d'ADN utilisant l'autoradiographie selon l'une quelconque des revendications 1 ou 2, dans lequel ladite feuille de matière phosphorescente stimulable comprend un support, une couche de matière phosphorescente comprenant une matière phosphorescente stimulable dispersée dans un liant, et une pellicule protectrice.

**Patentansprüche**

1. Ein Verfahren zum Bestimmen der Basensequenz von DNA oder eines DNA-Fragments unter Verwendung von Autoradiographie, das die Schritte umfaßt:
   (1) Herstellen einer Mischung von radioaktiv markierten, in vitro enzymatisch synthetisierten DNAs, die zu der zu sequenzierenden DNA oder dem DNA-Fragment komplementär sind;
   (2) Auftrennen der Mischung von in vitro enzymatisch synthetisierten DNAs auf einem Trägermedium;
   (3) Anbringen des Trägermediums und einer anregbaren Phosphorfolie in Schichten für eine gegebene Zeitspanne, um die Phosphorfolie zu veranlassen, mindestens einen Teil der Strahlungsenergie zu absorbieren, die von den radioaktiv markierten Substanzen auf dem Trägermedium emittiert wird, Anregen der Phosphorfolie mit einer elektromagnetischen Welle, um die in der Phosphorfolie gespeicherte Strahlungsenergie als angeregte Emission freizusetzen, und Nachweisen der angeregten Emission, um Information über die Position der radioaktiv markierten, in vitro enzymatisch synthetisierten DNAs zu erhalten; und
   (4) Bestimmen der Basensequenz der DNA oder des DNA-Fragments gemäß der erhaltenen Information über die Position.

2. Das Verfahren zum Bestimmen der Basensequenz einer DNA oder eines DNA-Fragments unter Verwendung von Autoradiographie wie in Anspruch 1 beansprucht, worin die in vitro enzymatisch synthetisierten DNAs in dem Schritt (1) solche sind, die durch die Dideoxykettenabbruchsmethode hergestellt worden sind.

3. Das Verfahren zum Bestimmen der Basensequenz einer DNA oder eines DNA-Fragments unter Verwendung von Autoradiographie wie in einem der Ansprüche 1 oder 2 beansprucht, worin die Mischung von in vitro enzymatisch synthetisierten DNAs in dem Schritt 2 aufgetrennt wird durch Gelelektrophorese.

4. Das Verfahren zum Bestimmen der Basensequenz von DNA oder eines DNA-Fragments unter Verwendung von Autoradiographie wie in einem der Ansprüche 1 oder 2 beansprucht, worin die anregbare Phosphorfolie in dem Schritt (3) angeregt wird durch Abtasten in Folge mit einer elektromagnetischen Welle.

5. Das Verfahren zum Bestimmen der Basensequenz von DNA oder eines DNA-Fragments unter Verwendung von Autoradiographie wie in einem der Ansprüche 1 oder 2 beansprucht, worin die Information über die Position der radioaktiv markierten Substanzen in dem Schritt (3) als ein Bild erhalten wird.

6. Das Verfahren zum Bestimmen der Basensequenz von DNA oder eines DNA-Fragments unter Verwendung von Autoradiographie wie in einem der Ansprüche 1 oder 2 beansprucht, worin die Information über die Position der radioaktiv markierten Substanzen in dem Schritt (3) in der Form von Symbolen und/oder Ziffern erhalten wird.

7. Das Verfahren zum Bestimmen der Basensequenz von DNA oder einem DNA-Fragment unter Verwendung von Autoradiographie wie in einem der Ansprüche 1 oder 2 beansprucht, worin die anregbare Phosphorfolie einen Träger, eine Phosphorschicht, enthaltend einen anregbaren Phosphor dispergiert in einem Bindemittel, und einen Schutzfilm umfaßt.

FIG.1